# EUROPÄISCHE PATENTSCHRIFT

(11) **EP 1 487 835 B1**
(45) Veröffentlichungstag und Bekanntmachung des Hinweises auf die Patenterteilung: **20.09.2006**
(21) Anmeldenummer: 03744337.1
(22) Anmeldetag: 03.03.2003
(51) Int. Cl.: C07D 453/02, A61K 31/439, A61P 25/28

(54) **ESSIG- UND PROPIONSÄUREAMIDE**
AMIDES OF ACETIC AND PROPIONIC ACIDS
AMIDES D'ACIDE ACETIQUE ET PROPIONIQUE

(30) Priorität: 15.03.2002 DE 10211416
(43) Veröffentlichungstag der Anmeldung: 22.12.2004
(73) Patentinhaber: Bayer HealthCare AG, 51368 Leverkusen (DE)
(72) Erfinder: LUITHLE, Joachim, 42489 Wülfrath (DE); BÖSS, Frank-Gerhard, Berkshire SL5, 7DF (GB); ERB, Christina, 65830 Kriftel (DE); SCHNITZLER, Katrin, 63517 Rodenbach (DE); FLESSNER, Timo, 42113 Wuppertal (DE); VAN KAMPEN, Marja, 63263 Neu-Isenburg (DE); METHFESSEL, Christoph, 42327 Wuppertal (DE)
(86) Internationale Anmeldenummer: PCT/EP2003/002152
(87) Internationale Veröffentlichungsnummer: WO 2003/078430

(56) Entgegenhaltungen:
- WO-A-01/02405
- WO-A-01/60821

## Beschreibung

Die Erfindung betrifft neue Essig- und Propionsäureamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi und Changeux, *Neuropharmacol.* **1995**, *34*, 563-582). Ein funktioneller nAChR besteht aus fünf Untereinheiten, die unterschiedlich (bestimmte Kombinationen von α1-9 und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee und Role, *Annu. Rev. Physiol.,* **1995,** *57*, 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani und Levin, *Biol. Psychiatry* **2001**, *49*, 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J. Neurosci.* **1993,** *13*, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide und Leslie, *Mol. Neurobiol.* **1999,** *20*, 1-16).

Bestimmte Chinuclidincarbonsäureanilide sind als Antiarrhythmika und Lokalanästhetika beschrieben (vgl. beispielsweise FR 1.566.045, GB 1 578 421 und Oppenheimer et al., *Life Sci*. **1991,** *48*, 977-985).

Die WO 01/60821 offenbart Biarylcarbonsäureamide mit Affinität zum α7-nAChR zur Behandlung von Lern- und Wahrnehmungsstörungen.

Die vorliegende Erfindung betrift Verbindungen der allgemeinen Formel (I) 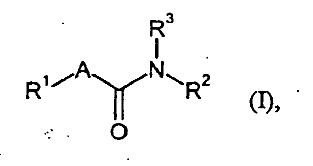 in welcher
- R¹: für einen 1-Aza-bicyclo[2.2.2]octyl- Rest steht,
welcher gegebenenfalls durch (C₁-C₆)-Alkyl substituiert ist,
- A: für Methylen oder Ethylen steht,
- R²: für Benzotriazolyl, Benzothiophenyl, Chinolinyl, Benzopyrazinyl Naphthyl oder Azulenyl steht, wobei die Ringe gegebenenfalls durch Reste ausgewählt aus der Gruppe Halogen, Formyl, -CO-NR⁴R⁵, -CO-OR⁶, -NR⁷R⁸, -NR⁹-CO-R¹⁰, Cyano, Trifluormethyl, Trifluormethoxy, Nitro, gegebenenfalls durch Hydroxyl, Amino, -NH-CO-R¹¹ oder Cyano substituiertes (C₁-C₆)-Alkyl, (C₁-C₆)-Alkoxy und (C₁-C₆)-Alkylthio substituiert sind,
worin R⁴, R⁵, R⁶, R⁷, R⁸, R⁹, R¹⁰ und R¹¹ unabhängig voneinander Wasserstoff, (C₁-C₆)-Alkyl, Phenyl oder Benzyl bedeuten,
und
- R³: für Wasserstoff oder (C₁-C₆)-Alkyl steht.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Solvate oder Solvate der Salze vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder N-Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfmdung haben die Substituenten im Allgemeinen die folgende Bedeutung:
(C₁-C₆)- und (C₁-C₄)-Alkoxy steht für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.-Butoxy, n-Pentoxy und n-Hexoxy.
(C₁-C₆)- und (C₁-C₄)-Alkyl stehen für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4, besonders bevorzugt mit 1 bis 3 Kohlenstoffatomen. Beispielsweise und vorzugsweise seien genannt: Methyl, Ethyl, n-Propyl, Isopropyl, tert.-Butyl, n-Pentyl und n-Hexyl.
(C₁-C₆)-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.-Butylthio, n-Pentylthio und n-Hexylthio.
Halogen steht für Fluor, Chlor, Brom und Iod. Bevorzugt sind Fluor, Chlor und Brom. Besonders bevorzugt sind Fluor und Chlor.
8- bis 10-gliedriges Heteroaryl steht für einen aromatischen, bicyclischen Rest mit 8 bis 10, vorzugsweise 9 bis 10 Ringatomen und bis zu 5, vorzugsweise bis zu 4 Heteroatomen aus der Reihe S, O und/oder N. Der Heteroarylrest kann über ein Kohlenstoff- oder Heteroatom gebunden sein. Beispielsweise und vorzugsweise seien genannt: Indolyl, Indazolyl, Benzofuranyl, Benzothiophenyl, Chinolinyl, Isochinolinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der allgemeinen Formel (I), in welcher
- R¹: für 1-Aza-bicyclo[2.2.2]octyl steht.

Besonders bevorzugt sind Verbindungen der allgemeinen Formel (I), in welcher
- R¹: für 1-Aza-bicyclo[2.2.2]oct-3-yl steht.

Ebenfalls bevorzugt sind Verbindungen der allgemeinen Formel (I), in welcher
- A: für Methylen steht.

Ebenfalls bevorzugt sind Verbindungen der allgemeinen Formel (I), in welcher
- R²: für Indolyl, Benzoimidazolyl, Benzotriazolyl, Benzothiophenyl, Benzofuranyl, Chinolinyl, Isochinolyl, Benzopyrazinyl, Benzopyrimidinyl, Benzopyridizanyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind.

Ganz besonders bevorzugt sind Verbindungen der allgemeinen Formel (I), in welcher
- R²: für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind.

Am meisten bevorzugt sind Verbindungen der allgemeinen Formel (I), in welcher
- R²: für Benzothiophen-2-yl, das gegebenenfalls durch 1 bis 3 Reste ausgewählt aus der Gruppe Wasserstoff, Halogen, Cyano, Trifluormethyl und C₁-C₄)-Alkyl substituiert ist, steht.

Ebenfalls bevorzugt sind Verbindungen der allgemeinen Formel (I), in welcher
- R³: für Wasserstoff oder Methyl steht.

Besonders bevorzugt sind Verbindungen der allgemeinen Formel (I), in welcher
- R³: für Wasserstoff steht.

Ganz besonders bevorzugt sind Kombinationen von zwei oder mehreren der oben genannten Vorzugsbereiche.

Ebenfalls ganz besonders bevorzugt sind Verbindungen der allgemeinen Formel (I), in welcher
- R¹: für 1-Aza-bicyclo[2.2.2]oct-3-yl steht,
- A: für Methylen steht,
- R²: für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und
- R³: für Wasserstoff steht.

Ebenfalls ganz besonders bevorzugt sind Verbindungen der allgemeinen Formel (I), in welcher
- R¹: für 1-Aza-bicyclo[2.2.2]oct-3-yl steht,
- A: für Methylen steht,
- R²: für Benzothiophenyl, Chinolinyl oder Naphthyl steht, wobei die Ringe gegebenenfalls durch 1 bis 2 Reste ausgewählt aus der Gruppe Wasserstoff, Fluor, Chlor, Brom, Nitro substituiert sind, und
- R³: für Wasserstoff steht, und deren Salze, Solvate und Solvate der Salze.

Die Erfindung betrifft weiterhin Verfahren zur Herstellung der Verbindungen der Formel (I), dadurch gekennzeichnet, dass man Verbindungen der allgemeinen Formel (II) 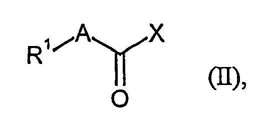 in welcher
- R¹ und A: die oben angegebene Bedeutung haben, und
- X: für Hydroxy oder eine geeignete Abgangsgruppe steht,
mit einer Verbindung der allgemeinen Formel (III)

R²R³NH (III),

in welcher R² und R³ die oben angegebene Bedeutung haben,
gegebenenfalls in einem inerten Lösungsmittel gegebenenfalls in Gegenwart eines Kondensationsmittels und gegebenenfalls in Gegenwart einer Base umsetzt,
und die resultierenden Verbindungen (I) gegebenenfalls mit den entsprechenden (i) Lösungsmitteln und/oder (ii) Basen oder Säuren zu ihren Solvaten, Salzen oder Solvaten der Salze umsetzt.

Wenn X eine Abgangsgruppe ist, sind Chlor, Mesyloxy, Isobutyloxycarbonyloxy, Pentafluorphenoxy oder Polymer-gebundenes 4-Carboxy-2,3,5,6-tetrafluorphenoxy, insbesondere Chlor bevorzugt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, Dimethylsulfoxid, Acetonitril oder Pyridin. Bevorzugt ist Dimethylformamid, Tetrahydrofuran, Methylenchlorid oder Chloroform.

Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformiat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetramethyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluroniumhexafluorophosphat (HATU) oder Benzotriazol-1-yloxytris(dimethylamino)-phosphoniumhexafluorophosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenzotriazol (HOBt) zu verwenden.

Besonders bevorzugt ist HATU oder die Kombination von N-(3-Dimethylaminoisopropyl)-N'-ethylcarbodiimid-Hydrochlorid (EDC) und 1-Hydroxybenzotriazol (HOBt) in Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise wird das erfindungsgemäße Verfahren in einem Temperaturbereich von Raumtemperatur bis +50°C bei Normaldruck durchgeführt.

Die Verbindungen der allgemeinen Formeln (II) und (III) sind bekannt oder lassen sich nach bekannten Verfahren aus den entsprechenden Edukten synthetisieren (vgl. z.B. Kato et al. *Chem. Pharm. Bull.* **1995,** *43*, 1351-1357).

So kann beispielsweise 1-Azabicyclo[2.2.2]oct-3-yl-essigsäure aus Chinuclidin-3-on durch Wittig-Horner-Reaktion und nachfolgende Hydrierung und Ester-Hydrolyse gemäß folgendem Syntheseschema erhalten werden:

### Syntheseschema

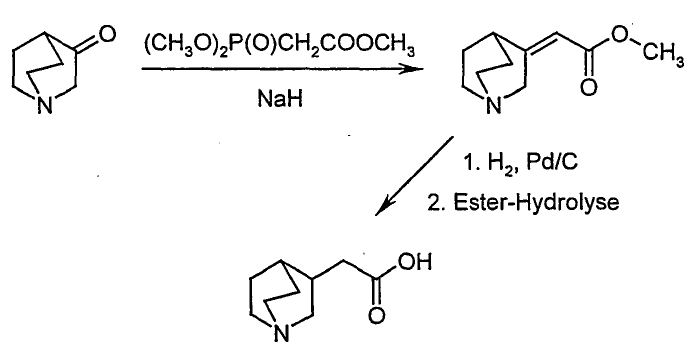

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie zeichnen sich als Liganden, insbesondere Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen, insbesondere der Alzheimerschen Krankheit eingesetzt werden. Wegen ihrer selektiven Wirkung als a7-nAChR-Agonisten eignen sich die erfindungsgemäßen Verbindungen besonders zur Verbesserung der Wahrnehmung, Konzentrationsleistung, Lernleistung oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen in Kindern mit Lern- und Gedächtnisproblemen, Attention Deficit Hyperactivity Disorder, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrophe Lateralsklerose (ALS), Huntingtonsche Krankheit, Multiple Sklerose, Thalamische Degeneration, Creutzfeld-Jacob-Demenz, HIV-Demenz, Schizophrenie, Schizophrenie mit Demenz oder Korsakoff-Psychose.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994), insbesondere zur Behandlung von Krebs-induzierten Schmerzen und chronischen neuropathischen Schmerzen, wie zum Beispiel, bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die erfindungsgemäßen Verbindungen können allein oder in Kombination mit anderen Wirkstoffen zur Behandlung akuter oder chronischer neurodegenerativer Erkrankungen eingesetzt werden, wie zum Beispiel Schlaganfall, Schädel-Hirn-Trauma, Rückenmarksverletzungen, Parkinson'sche Krankheit, Huntington'sche Krankheit, Alzheimer'sche Krankheit, Multiple Sklerose, Amyotrophe Lateralsklerose (ALS) und Niemann Pick Krankheit.

Die *in vitro*-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine Bindungstest ist eine Modifikation der von Davies et al. (*Neuropharmacol*. **1999**, *38*, 679-690) beschriebenen Methode.

Rattenhirngewebe (Hippocampus oder Gesamthirn) wird in Homogenisierungspuffer (10% w/v) [0.32 M Sucrose, 1 mM EDTA, 0.1 mM Phenylmethylsulfonylfluorid (PMSF), 0.01% (w/v) NaN₃, pH 7.4, 4°C] bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20% w/v) und zentrifugiert (1000 x g, 4°C, 10 min). Die beiden Überstände werden vereinigt und zentrifugiert (15.000 x g, 4°C, 30 min). Dieses Pellet wird als P2-Fraktion bezeichnet.

Das P2-Pellet wird zweimal mit Bindungspuffer gewaschen (50 mM Tris-HCl, 1 mM MgCl₂, 120 mM NaCl, 5 mM KCl, 2 mM CaCl₂, pH 7.4) und zentrifugiert (15.000 x g, 4°C, 30 min).

Die P2-Membranen werden in Bindungspuffer resuspendiert und in einem Volumen von 250 µl (Membranproteinmenge 0.1 - 0.5 mg) für 2.5 h bei 21°C inkubiert in der Gegenwart von 1-5 nM [³H]-Methyllycaconitine, 0.1% (w/v) BSA (bovines Serumalbumin) und verschiedenen Konzentrationen der Testsubstanz. Die unspezifische Bindung wird bestimmt durch Inkubation in der Gegenwart von 1 µM α-Bungarotoxin oder 100 µM Nicotin oder 10 µM MLA (Methyllycaconitine).

Die Inkubation wird beendet durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3% (v/v) Polyethyleneimine (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50% des am Rezeptor gebundenen Liganden verdrängt werden), der Dissoziationskonstante K_{D} und der Konzentration L von [³H]-Methyllycaconitine wird die Dissoziationskonstante der Testsubstanz Ki bestimmt (Kᵢ = IC₅₀ / (1+L/K_{D})).

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Die in-vitro-Wirkdaten für die erfindungsgemäßen Verbindungen sind in Tabelle A wiedergegeben:

**Tabelle A**

| **Beispiel Nr.** | **K**_{**i**}**-Wert [nM]** |
|---|---|
| 1 | 120 |
| 2 | 200 |
| 3 | 280 |
| 4 | 42 |
| 5 | 170 |

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al. *NeuroReport* **1998,** *9,* 4205-4208; Ennaceur, A., Delacour, J.,. *Behav. Brain Res.* **1988,** *31,* 47-59; Ennaceur, A., Meliani, K.,. *Psychopharmacology* **1992,** *109,* 321-330; Prickaerts, et al. *Eur. J. Pharmacol.* **1997**, *337*, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtrungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern-und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskiminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest

Der Soziale Wiedererkennungstest ist ein Test zur Prüfung der lern- oder gedächtnisverbessernden Wirkung von Testsubstanzen.

Erwachsene Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die totale Zeit gemessen, die das adulte Tier das Junge investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das Juvenile herausgenommen und das Adulte in seinem Testkäfig belassen (bei 24 Stunden Retention wird das Tier in seinen Heimkäfig zurückgesetzt). Vor oder nach dem ersten Test wird das Versuchstier mit Substanz behandelt. Je nach Zeitpunkt der Substanzgabe wird das Erlernen oder das Speichern der Information über das Jungtier durch die Substanz beeinflusst. Nach einem festgelegten Zeitraum (Retention) wird der Test wiederholt (Trial 2). Je größer die Differenz zwischen den in Trial 1 und 2 ermittelten Investigationszeiten, desto besser hat sich das adulte Tier an das Jungtier erinnert

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dein oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DCI: direkte chemische Ionisation (bei MS)
- DCM: Dichlormethan
- DMAP: 4-*N,N*-Dimethylaminopyridin
- DMF: *N,N*-Dimethylformamid
- DMSO: Dimethylsulfoxid
- d.Th.: der Theorie (bei Ausbeute)
- EDC: *N*'-(3-Dimethylaminopropyl)-*N*-ethylcarbodiimid x HCl
- ESI: Elektrospray-Ionisation (bei MS)
- HATU: *O*-(7-Azabenzotriazol-1-yl)-*N*,*N*,*N*',*N*'-tetramethyluronium-Hexafluorophosphat
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- PS: Polystyrol (-Harz)
- RT: Raumtemperatur
- Rₜ: Retentionszeit (bei HPLC)
- THF: Tetrahydrofuran

### HPLC-Methoden:

### Methode 1:

Instrument: HP 1100 mit DAD-Detektion; Säule: Kromasil RP-18, 60 mm x 2 mm, 3.5 µm; Eluent A: 5 ml HClO₄/L H₂O, Eluent B: Acetonitril; Gradient: 0 min 2% B, 0.5 min 2% B, 4.5 min 90% B, 6.5 min 90% B; Fluss: 0.75 mL/min; Temperatur: 30°C; Detektion: UV 210 nm.

### Methode 2:

Säule: Kromasil 100 C-18, 125 mm x 3 mm, 5 µm; Eluent A: 0.2% HClO₄, Eluent B: Acetonitril; Gradient: 0 min 5% B, 5 min 95% B; Fluss: 1.25 mL/min; Temperatur: 40°C; Detektion: UV 210 nm.

### Ausgangsverbindungen:

### Beispiel 1A

### Chinuclidin-3-on

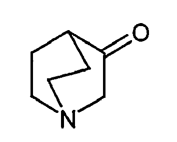

100 g (0.62 mol) Chinuclidin-3-on-Hydrochlorid werden in 2 L Methanol suspendiert. Bei 0°C wird eine Lösung von 33.4 g (0.62 mol) Natriummethylat in 250 mL Methanol langsam zugetropft. Es wird 16 h bei Raumtemperatur nachgerührt. Der entstandene Niederschlag wird abgesaugt, das Filtrat wird im Vakuum eingeengt. Der Rückstand wird zwischen Chloroform und Wasser verteilt und mit Chloroform extrahiert. Die vereinigten organischen Phasen werden über Natriumsulfat getrocknet und im Vakuum eingeengt. Man erhält 58.8 g (75.9% d.Th.) der Titelverbindung.
MS (DCI): *m*/*z* = 126 (M+H)⁺, 143 (M+NH₄)⁺
¹H-NMR (300 MHz, CDCl₃): δ = 3.30 (m, 2H), 3.19-2.86 (m, 4H), 2.46 (m, 1H), 1.99 (m, 4H).

### Beispiel 2A

### Methyl (2Z)-1-azabicyclo[2.2.2]oct-3-yliden-ethanoat-Hydrochlorid

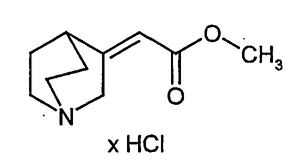

25.3 g (0.63 mol) Natriumhydrid (als 60%-ige Suspension in Mineralöl) werden in 480 mL Dimethylformamid suspendiert. Nach dem Hinzutropfen einer Lösung von 104.8 g (0.58 mol) Phosphonoessigsäuretrimethylester in 480 mL Dimethylformamid wird bis zur Beendigung der Wasserstoffentwicklung bei Raumtemperatur nachgerührt. Eine Lösung von 36 g (0.29 mol) Chinuclidin-3-on in 480 mL Dimethylformamid wird über einen Zeitraum von 40 Minuten hinzugetropft und anschließend für 16 h bei Raumtemperatur nachgerührt. Das Reaktionsgemisch wird im Vakuum eingeengt, der Rückstand zwischen Wasser und Essigsäureethylester verteilt und mit Essigsäureethylester extrahiert. Die vereinigten organischen Phasen werden über Natriumsulfat getrocknet und im Vakuum eingeengt. Der Rückstand wird an Kieselgel säulenchromatographisch gereinigt (Laufmittel: Dichlormethan / Methanol Ammoniak = 95:5:0.5). Das erneut eingeengte Material wird in wenig Dichlormethan gelöst und mit etherischer HCl versetzt. Der entstehende Niederschlag wird abgesaugt und mit Diethylether nachgewaschen. Nach der Trocknung bei 35°C werden 19.53 g (31.2% d.Th.) der Titelverbindung in Form weißer Kristalle erhalten. HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ = 2.40 min.
MS (DCI): *m*/*z* = 182 (M+H)⁺, 199 (M+NH₄)⁺, 363 (2M+H)⁺
¹H-NMR (500 MHz, DMSO-d₆): δ = 11.56 (breites s, 1H), 5.97 (m, 1H), 4.32 (m, 2H), 3.66 (s, 3H), 3.27 (m, 4H), 2.84 (m, 1H), 2.13-1.92 (m, 2H), 1.91-1.69 (m, 2H); ¹³C-NMR (125 MHz, DMSO-d₆): δ = 165.72, 155.95, 113.08, 53.55, 51.28, 45.29, 30.14, 22.41.

### Beispiel 3A

### 1-Azabicyclo[2.2.2]oct-3-yl-essigsäure-Hydrochlorid

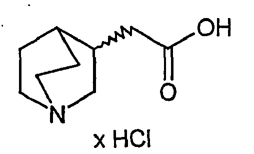

13.5 g (62 mmol) Methyl (2Z)-1-azabicyclo[2.2.2]oct-3-yliden-ethanoat werden in 200 mL Methanol gelöst und unter Argon mit 1 g Palladium auf Aktivkohle (10%-ig) versetzt. Bei Raumtemperatur wird das Reaktionsgemisch für 16 h unter Wasserstoffatmosphäre (Normaldruck) gerührt. Es wird über Kieselgur filtriert und mit Methanol nachgewaschen. Das Filtrat wird mit 50 mL 1 N Salzsäure versetzt, im Vakuum eingeengt und im Hochvakuum getrocknet. Der Rückstand wird in 100 mL 32%-iger Salzsäure für 5 h am Rückfluss erhitzt. Es wird im Vakuum eingeengt, zweimal mit Toluol codestilliert und im Hochvakuum getrocknet. Man erhält 11.8 g des Produkts in einer Reinheit von 89% (77% d.Th.).
HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ= 0.80 min.
MS (DCI): *m*/*z* = 170 (M+H)⁺, 339 (2M+H)⁺
¹H-NMR (200 MHz, DMSO-d₆): δ = 12.32 (breites s, 1H), 10.61 (s, 1H), 3.38 (m, 1H), 3.14 (m, 4H), 2.76 (dd, 1H), 2.67-2.22 (m, 4H), 2.01-1.55 (m, 4H).

### Beispiel 4A

### 6-Brom-1-benzothiophen-2-carbonsäuremethylester

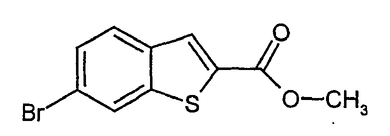

Zu einer Suspension von 1.93 g (48.3 mmol) Natriumhydrid (als 60%-ige Suspension in Mineralöl) in 65 mL DMSO werden bei Raumtemperatur langsam 3.76 g (35.5 mmol) Mercaptoessigsäuremethylester hinzugetropft. Nach Beendigung der Wasserstoffentwicklung gibt man eine Lösung von 6.54 g (32.2 mmol) 4-Brom-2-fluorbenzaldehyd in 10 mL DMSO hinzu. Nach 10 min. rührt man das Reaktionsgemisch in 200 mL Eiswasser ein und isoliert den entstehenden Niederschlag. Der Feststoff wird zweimal mit Wasser gewaschen und bei 50°C im Vakuum getrocknet. Man erhält 4.06 g (46.4% d.Th) der Titelverbindung.
¹H-NMR (200 MHz, DMSO-d₆): δ = 8.42 (d, 1H), 8.22 (s, 1H), 7.98 (d, 1H), 7.65 (dd, 1H), 3.90 (s, 3H).
HPLC (Methode 1): Rₜ = 5.3 min.
MS (ESIpos): *m*/*z* = 270 (M⁺).

### Beispiel 5A

### 6-Brom-1-benzothiophen-2-carbonsäure

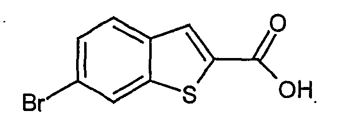

Eine Lösung von 4.0 g (14.8 mmol) 6-Brom-1-benzothiophen-2-carbonsäuremethylester in 40 mL einer 1:1-Mischung aus THF und 2 N Kalilauge wird 2 h bei Raumtemperatur gerührt. Das Lösungsmittel wird im Vakuum entfernt, und der Rückstand wird mit konzentrierter Salzsäure angesäuert. Der entstehende Niederschlag wird abgesaugt, mit Wasser nachgewaschen und bei 50°C im Vakuum getrocknet. Es werden 3.55 g (93.5% d.Th.) des gewünschten Produkts erhalten.
¹H-NMR (400 MHz, DMSO-d₆): δ = 13.48 (breites s, 1H), 8.38 (s, 1H), 8.22 (s, 1H), 7.96 (d, 1H), 7.63 (m, 1H).
HPLC (Methode 1): Rₜ = 4.5 min.

### Beispiel 6A

### 6-Brom-1-benzothiophen-2-amin-Hydrochlorid

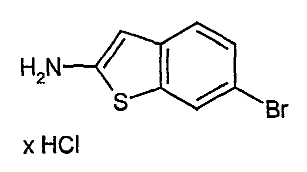

Zu einer Lösung von 1 g (3.89 mmol) 6-Brom-1-benzothiophen-2-carbonsäure und 1.35 mL (7.78 mmol) N,N-Diisopropylethylamin in 10 mL DMF werden bei 0°C 0.92 mL (4.28 mmol) Diphenylphosphorazidat hinzugefügt. Nach 2 h bei 0°C wird das Reaktionsgemisch auf Eiswasser gegeben und mit Essigsäure neutralisiert. Der entstehende Niederschlag wird abgesaugt und mit Wasser nachgewaschen. Der noch feuchte Feststoff wird in 5 mL Xylol suspendiert, in 1 mL siedendes tert.-Butanol getropft und 3 h am Rückfluss erhitzt. Nach dem Abkühlen wird das Lösungsmittel im Vakuum entfernt. Der Rückstand wird in 4 M HCl in Dioxan gelöst und 1 h bei Raumtemperatur gerührt. Der entstehende Niederschlag wird abgesaugt und im Vakuum getrocknet. Man erhält 294 mg (28.1% d.Th.) der Titelverbindung.
HPLC (Methode 1): Rₜ = 5.5 min.
MS (ESIpos): *m*/*z* = 228 (M+H)⁺ (freie Base).

### Beispiel 7A

### (1-Azabicyclo[2.2.2]oct-3-yl)-essigsäure-pentafluorphenylester-Hydrochlorid

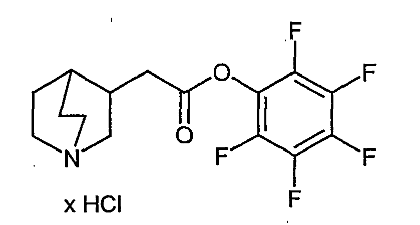

Zu einer Lösung aus 100 mg (0.49 mmol) 1-Azabicyclo[2.2.2]oct-3-yl-essigsäure-Hydrochlorid in 4 mL Dichlormethan werden bei 0°C 358 mg (1.94 mmol) Pentafluorphenol, 120.5 mg (0.63 mmol) EDC sowie ein Tropfen N,N-Diisopropylethylamin hinzugefügt. Bei Raumtemperatur wird für 18 h gerührt. Der Kolbeninhalt wird im Vakuum eingeengt und im Hochvakuum getrocknet. Das erhaltene Rohprodukt wird ohne weitere Reinigung in den Folgestufen eingesetzt.

### Beispiel 8A

### an polymeres Trägerharz gebundene 4-Hydroxy-2,3,5,6-tetrafluorbenzoesäure

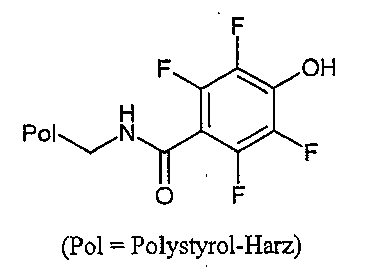

51.2 g Polystyrol-Aminomethyl-Harz (Beladung 1.36 mmol/g, 69.6 mmol; Fa. Argonaut Technologies, USA) werden in 700 mL DMF suspendiert. Es werden 41.6 g (107.8 mmol) HOBt und 24.1 g (114.8 mmol) 4-Hydroxy-2,3,5,6-tetrafluorbenzoesäure zugegeben. Das Reaktionsgemisch wird unter gelindem Rühren nach 15 min mit 16.9 mL (107.8 mmol) N,N'-Diisopropylcarbodiimid versetzt, und anschließend wird über Nacht gerührt. Es wird filtriert, und das zurückbleibende Harz wird mit DMF nachgewaschen. Das erhaltene Harz wird in 450 mL DMF resuspendiert, mit 8.26 mL (83.5 mmol) Piperidin versetzt und geschüttelt. Nach 2 h wird erneut filriert und das verbleibende Harz in eine Lösung von 120 mL 1 M Salzsäure in 500 mL DMF gegeben und weitere 2 h geschüttelt. Nach erneuter Filtration wird mit jeweils 500 mL DMF, THF und DCM nachgewaschen. Nach Trocknen bei 50°C im Vakuum werden 77.2 g der Polymer-gebundenen Titelverbindung erhalten.

### Beispiel 9A

### an polymeres Trägerharz gebundene 4-{2-(1-Azabicyclo[2.2.2]oct-3-yl)-acetoxy}-2,3,5,6-tetrafluorbenzoesäure

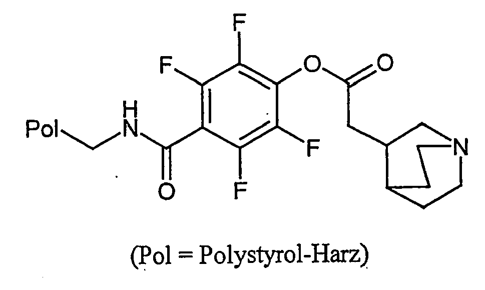

2 g des polymeren Trägerharzes aus Beispiel 8A (Beladung ca. 1.36 mmol/g, 2.72 mmol) werden in 20 mL DMF suspendiert und mit 1.23 g (5.98 mmol) 1-Azabicyclo[2.2.2]oct-3-yl-essigsäure-Hydrochlorid und 130 mg (1.09 mmol) DMAP 10 min lang geschüttelt. Anschließend werden 1.06 mL (6.80 mmol) N,N'-Diisopropylcarbodiimid zugegeben und über Nacht geschüttelt. Das Harz wird abgesaugt, jeweils zweimal mit je 20 mL DMF, THF und DCM nachgewaschen und im Hochvakuum getrocknet. Es werden 2.318 g der Polymer-gebundenen Titelverbindung erhalten.

### Ausführungsbeispiele:

### Beispiel 1

### 2-(1-Azabicyclo[2.2.2]oct-3-yl)-N-(7-brom-1-benzothien-2-yl)-acetamid-Hydrochlorid

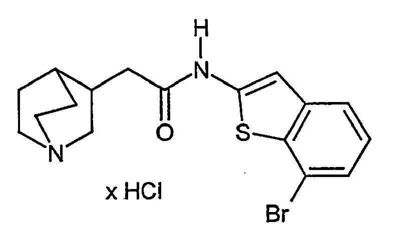

162.3 mg (0.79 mmol) der racemischen 1-Azabicyclo[2.2.2]oct-3-yl-essigsäure werden zusammen mit 120 mg (0.53 mmol) 3-Brom-1-benzothiophen-2-amin und 300.0 mg (0.79 mmol) HATU bei 0°C in DMF vorgelegt. Nach Zugabe von 102.0 mg (0.79 mmol) N,N-Diisopropylethylamin wird 30 min. gerührt. Es werden weitere 204.0 mg (1.58 mmol) N,N-Diisopropylethylamin zugegeben und bei RT über Nacht nachgerührt. Die Reinigung erfolgt durch präparative HPLC. Das Produkt wird in wenig Acetonitril gelöst und mit einem Überschuß an 1 N etherischer HCl versetzt. Das Solvens wird im Vakuum abgezogen. Es werden 12 mg (5% d.Th.) der Titelverbindung erhalten.
HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp:: 30°C; UV-Detektion bei 210 nm): Rₜ = 4.20 min.
MS (ESIpos): *m*/*z* = 379 (M+H)⁺ (freie Base).

### Beispiel 2

### 2-(1-Azabicyclo[2.2.2]oct-3-yl)-N-(6-brom-1-benzothien-2-yl)acetamid-Hydrochlorid

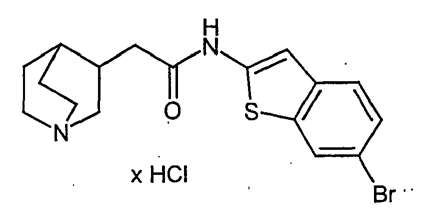

89.2 mg (0.24 mmol) (1-Azabicyclo[2.2.2]oct-3-yl)-essigsäure-pentafluorphenyl-, ester-Hydrochlorid werden in 1 mL DMF gelöst, mit 71.2 mg (0.31 mmol) 6-Brom-1-benzothiophen-2-amin versetzt und über Nacht bei Raumtemperatur gerührt. Es wird 1 g MP-Carbonat (Polymer-gebundenes Carbonat, Kapazität: 2.5 - 3.5 mmol/g; Fa. Argonaut Technologies, USA) zugegeben. Nach 3 h wird das Polystyrol-Harz abfiltriert und mit THF nachgewaschen. Die vereinigten Filtrate werden im Vakuum eingeengt und das Rohprodukt über präparative HPLC gereinigt. Zur Hydrochlorid-Herstellung wird das Produkt mit einer Mischung aus 1 M Salzsäure und Acetonitril versetzt und erneut eingeengt. Nach Trocknen im Hochvakuum erhält man 14 mg (14% d.Th.) der Titelverbindung.
HPLC (Methode 1): Rₜ = 4.2 min.
MS (ESIpos): *m*/*z* = 379 (M+H)⁺ (freie Base).

### Beispiel 3

### 2-(1-Azabicyclo[2.2.2]oct-3-yl)-N-(7-chinolinyl)acetamid-Hydrochlorid

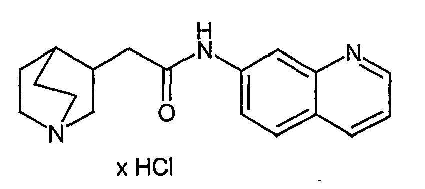

90.3 mg (0.24 mmol) (1-Azabicyclo[2.2.2]oct-3-yl)-essigsäure-pentafluorphenylester-Hydrochlorid werden in 1 mL DMF gelöst, mit 52.6 mg (0.36 mmol) 6-Aminochinolin versetzt und über Nacht bei Raumtemperatur gerührt. Es wird 1 g MP-Carbonat (Polymer-gebundenes Carbonat, Kapazität: 2.5 - 3.5 mmol/g; Fa. Argonaut Technologies, USA) zugegeben. Nach 1 h wird das Polystyrol-Harz abfiltriert und mit THF nachgewaschen. Die vereinigten Filtrate werden im Vakuum eingeengt und das Rohprodukt über präparative HPLC gereinigt. Zur Hydrochlorid-Herstellung wird das Produkt mit einer Mischung aus 1 M Salzsäure und Acetonitril versetzt und erneut eingeengt. Nach Trocknen im Hochvakuum erhält man 44 mg (50.2% d.Th.) der Titelverbindung.
HPLC (Methode 2): Rₜ = 2.8 min.
MS (DCI): *m*/*z* = 296 (M+H)⁺ (freie Base).

### Beispiel 4

### 2-(1-Azabicyclo[2.2.2]oct-3-yl)-N-(2-naphthyl)acetamid-Hydrochlorid

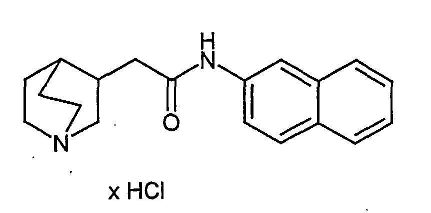

500 mg des polymeren Trägerharzes aus Beispiel 9A (Beladung ca. 1.36 mmol/g, 0.68 mmol) werden in 5 mL DMF suspendiert, mit 77.9 mg (0.54 mmol) 2-Aminonaphthylamin versetzt und 2 Tage bei Raumtemperatur geschüttelt. Das Harz wird abgesaugt und jeweils zweimal mit THF und DMF nachgewaschen. Die vereinigten Filtrate werden im Vakuum eingeengt. Der Rückstand wird in Methanol aufgenommen, mit etwas Palladium auf Aktivkohle (10%-ig) versetzt und über Nacht bei Normaldruck hydriert. Der Katalysator wird über Kieselgur abfiltriert und mit Methanol nachgewaschen. Der nach dem Einengen erhaltene Rückstand der vereinigten Methanol-Filtrate wird mittels präparativer HPLC gereinigt. Die vereinigten Produkt-Fraktionen werden mit 1 M Salzsäure versetzt und eingeengt. Nach Trocknen im Hochvakuum erhält man 13 mg (4.75% d.Th.) der Titelverbindung.
HPLC (Methode 1): Rₜ = 3.9 min.
MS (ESIpos): *m*/*z* = 295 (M+H)⁺ (freie Base).

### Beispiel 5

### 2-(1-Azabicyclo[2.2.2]oct-3-yl)-N-(8-nitro-2-naphthyl)acetamid-Hydrochlorid

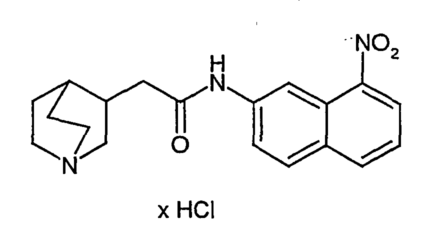

200 mg (0.97 mmol) 1-Azabicyclo[2.2.2]oct-3-yl-essigsäure-Hydrochlorid werden 2 h lang in 2 mL (27.42 mmol) Thionylchlorid unter Rückfluss erhitzt. Anschließend wird das Gemisch im Vakuum vom überschüssigen Thionylchlorid befreit und der Rückstand in 4 mL DMF aufgenommen. Zu dieser Lösung werden 0.54 mL (3.89 mmol) Triethylamin, 59.4 mg (0.4 mmol) DMAP und 183.0 mg (0.97 mmol) 8-Nitro-2-naphthylamin gegeben. Nach Reaktion über Nacht und Reinigung mittels präparativer HPLC werden die erhaltenen Produkt-Fraktionen mit 1 M Salzsäure versetzt und im Vakuum eingeengt. Nach Umkristallisation des Rückstandes aus Isopropanol und Trocknen im Hochvakuum erhält man 59 mg (15.9% d.Th.) der Titelverbindung.
HPLC (Methode 1): Rₜ = 3.9 min.
MS (ESIpos): *m*/*z* = 340 (M+H)⁺ (freie Base).

## Patentansprüche

1. Verbindungen der allgemeinen Formel (I) 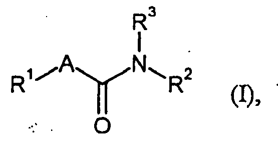 in welcher
R¹ für einen 1-Aza-bicyclo[2.2.2]octyl-Rest steht,
welcher gegebenenfalls durch (C₁-C₆)-Alkyl substituiert ist,
A für Methylen oder Ethylen steht,
R² für Benzotriazolyl, Benzothiophenyl, Chinolinyl, Benzopyrazinyl, Naphthyl oder Azulenyl steht, wobei die Ringe gegebenenfalls durch Reste ausgewählt aus der Gruppe Halogen, Formyl, -CO-NR⁴R⁵, -CO-OR⁶, -NR⁷R⁸, -NR⁹-CO-R¹⁰, Cyano, Trifluormethyl, Trifluormethoxy, Nitro, gegebenenfalls durch Hydroxyl, Amino, -NH-CO-R¹¹ oder Cyano substituiertes (C₁-C₆)-Alkyl, (C₁-C₆)-Alkoxy und (C₁-C₆)-Alkylthio substituiert sind, worin R⁴, R⁵, R⁶, R⁷, R⁸, R⁹, R¹⁰ und R¹¹ unabhängig voneinander: Wasserstoff, (C₁-C₆)-Alkyl, Phenyl oder Benzyl bedeuten,
und
R³ für Wasserstoff oder (C₁-C₆)-Alkyl steht,
und deren Salze, Solvate und Solvate der Salze.

2. Verbindungen nach Anspruch 1, wobei
R¹ für 1-Aza-bicyclo[2.2.2]oct-3-yl steht,
und R² und R³ die in Anspruch 1 angegebene Bedeutung haben,
und deren Salze, Solvate und Solvate der Salze.

3. Verbindungen nach Anspruch 1 oder 2, wobei
R¹ für 1-Aza-bicyclo[2.2.2]oct-3-yl steht,
A für Methylen steht,
R² für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und
R³ für Wasserstoff steht,
und deren Salze, Solvate und Solvate der Salze.

4. Verbindungen nach Ansprüchen 1, 2 oder 3, wobei
R¹ für 1-Aza-bicyclo[2.2.2]oct-3-yl steht,
A für Methylen steht,
R² für Benzothiophenyl, Chinolinyl oder Naphthyl steht, wobei die Ringe gegebenenfalls durch 1 bis 2 Reste ausgewählt aus der Gruppe Wasserstoff, Fluor, Chlor, Brom, Nitro substituiert sind,
und
R³ für Wasserstoff steht,
und deren Salze, Solvate und Solvate der Salze.

5. Verfahren zur Herstellung von Verbindungen der allgemeinen Formel (I) nach Anspruch 1, **dadurch gekennzeichnet, dass** man Verbindungen der allgemeinen Formel (II), 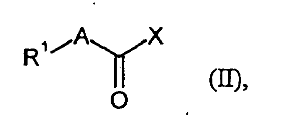 in welcher
R¹ und A die in Anspruch 1 angegebene Bedeutung haben, und
X für Hydroxy oder eine geeignete Abgangsgruppe steht,
mit einer Verbindung der allgemeinen Formel (III)
R²R³NH (III),
in welcher R² und R³ die in Anspruch 1 angegebene Bedeutung haben,
umsetzt und die resultierenden Verbindungen (I) gegebenenfalls mit den entsprechenden (i) Lösungsmitteln und/oder (ii) Basen oder Säuren zu ihren Solvaten, Salzen oder Solvaten der Salze umsetzt.

6. Verbindungen nach einem der Ansprüche 1 bis 4 zur Behandlung und/oder Prophylaxe von Krankheiten.

7. Arzneimittel enthaltend mindestens eine der Verbindungen nach einem der Ansprüche 1 bis 4 in Zusammenmischung mit mindestens einem pharmazeutisch verträglichen, im wesentlichen nichtgiftigen Träger oder Exzipienten.

8. Verwendung, von Verbindungen nach einem der Ansprüche 1 bis 4 zur Herstellung eines Arzneimittels zur Verbesserung der Wahrnehmung, Konzentrationsleistung, Lernleistung und/oder Gedächtnisleistung.

9. Verwendung von Verbindungen nach einem der Ansprüche 1 bis 4 zur Herstellung eines Arzneimittels zur Behandlung und/oder Prophylaxe von Störungen der Wahrnehmung, Konzentrationsleistung, Lernleistung und/oder Gedächtnisleistung.

10. Arzneimittel nach Anspruch 7 zur Behandlung und/oder Prophylaxe von Störungen der Wahrnehmung, Konzentrationsleistung, Lernleistung und/oder Gedächtnisleistung.

## Claims

1. Compound of the formula (I) 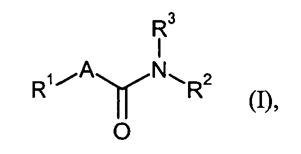 in which
R¹ is a 1-azabicyclo[2.2.2]octyl radical,
which is optionally substituted by (C₁-C₆)-alkyl,
A is methylene or ethylene,
R² is benzotriazolyl, benzothiophenyl, quinolinyl, benzopyrazinyl, naphthyl or azulenyl, where the rings are optionally substituted by radicals selected from the group of halogen, formyl, -CO-NR⁴R⁵, -CO-OR⁶, -NR⁷R⁸, -NR⁹-CO-R¹⁰, cyano, trifluoromethyl, trifluoromethoxy, nitro, optionally hydroxyl-, amino-, -NH-CO-R¹¹- or cyano-substituted (C₁-C₆)-alkyl, (C₁-C₆)-alkoxy, (C₁-C₆)-alkylthio, in which R⁴, R⁵, R⁶, R⁷, R⁸, R⁹, R¹⁰ and R¹¹ are independently of one another hydrogen, (C₁-C₆)-alkyl, phenyl or benzyl,
and
R³ is hydrogen or (C₁-C₆)-alkyl,
and the salts, solvates and solvates of the salts thereof.

2. Compound according to Claim 1, where
R¹ is 1-azabicyclo[2.2.2]oct-3-yl,
and R² and R³ have the meaning indicated in Claim 1,
and the salts, solvates and solvates of the salts thereof.

3. Compound according to Claim 1 or 2, where
R¹ is 1-azabicyclo[2.2.2]oct-3-yl,
A is methylene,
R² is benzotriazolyl, benzothiophenyl, quinolinyl, benzopyrazinyl or naphthyl, where the rings are optionally substituted by 1 to 3 radicals selected from the group of hydrogen, halogen, cyano, trifluoromethyl, trifluoromethoxy, nitro, (C₁-C₄)-alkyl, (C₁-C₄)-alkoxy, (C₁-C₄)-alkylthio,
and
R³ is hydrogen,
and the salts, solvates and solvates of the salts thereof.

4. Compound according to Claims 1, 2 or 3, where
R¹ is 1-azabicyclo[2.2.2]oct-3-yl,
A is methylene,
R² is benzothiophenyl, quinolinyl or naphthyl where the rings are optionally substituted by 1 to 2 radicals selected from the group of hydrogen, fluorine, chlorine, bromine, nitro
and
R³ is hydrogen,
and the salts, solvates and solvates of the salts thereof.

5. Process for preparing compounds of the general formula (I) according to Claim 1, **characterized in that** compounds of the general formula (II) 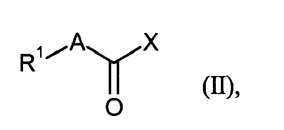 in which
R¹ and A have the meaning indicated in Claim 1, and
X is hydroxy or a suitable leaving group,
are reacted with a compound of the general formula (III)
R²R³NH (III),
in which R² and R³ have the meaning indicated in Claim 1,
and the resulting compounds (I) are converted where appropriate with the appropriate (i) solvents and/or (ii) bases or acids into the solvates, salts or solvates of the salts thereof.

6. Compound according to any of Claims 1 to 4 for the treatment and/or prophylaxis of diseases.

7. Medicament comprising at least one of the compounds according to any of Claims 1 to 4 mixed with at least one pharmaceutically acceptable, essentially nontoxic carrier or excipient.

8. Use of compounds according to any of Claims 1 to 4 for producing a medicament for improving perception, concentration, learning and/or memory.

9. Use of compounds according to any of Claims 1 to 4 for producing a medicament for the treatment and/or prophylaxis of impairments of perception, concentration, learning and/or memory.

10. Medicament according to Claim 7 for the treatment and/or prophylaxis of impairments of perception, concentration, learning and/or memory.

## Revendications

1. Composés de formule générale (I) 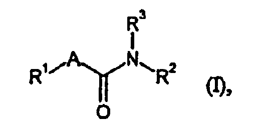 dans laquelle
R¹ représente un reste 1-aza-bicyclo[2.2.2]octyle, qui est éventuellement substitué par un radical alkyle en C₁ à C₆,
A représente un groupe méthylène ou éthylène,
R² représente un noyau benzotriazolyle, benzothiophényle, quinolinyle, benzopyrazinyle, naphtyle ou azulényle, les noyaux étant éventuellement substitués par des restes choisis dans le groupe des restes halogéno, formyle, -CO-NR⁴R⁵, -CO-OR⁶, -NR⁷R⁸, -NR⁹-CO-R¹⁰, cyano, trifluorométhyle, trifluorométhoxy, nitro, alkyle en C₁ à C₆ éventuellement substitué par un radical hydroxyle, amino, -NH-CO-R¹¹ ou cyano, un reste alkoxy en C₁ à C₆ et alkylthio en C₁ à C₆, où R⁴, R⁵, R⁶, R⁷, R⁸, R⁹, R¹⁰ et R¹¹ désignent, indépendamment les uns des autres, l'hydrogène, un radical alkyle en C₁ à C₆, phényle ou benzyle,
et
R³ représente l'hydrogène ou un reste alkyle en C₁ à C₆,
et leurs sels, leurs produits de solvatation et des produits de solvatation des sels.

2. Composés suivant la revendication 1, dans lesquels
R¹ est un reste 1-aza-bicyclo[2.2.2]oct-3-yle,
et R² et R³ ont la définition indiquée dans la revendication 1,
et leurs sels, leurs produits de solvatation et les produits de solvatation des sels.

3. Composés suivant la revendication 1 ou 2, dans lesquels
R¹ est un reste 1-aza-bicyclo[2.2.2]oct-3-yle,
A est un groupe méthylène,
R² est un noyau benzotriazolyle, benzothiophényle, quinolinyle, benzopyrazinyle ou naphtyle, les noyaux étant éventuellement substitués par 1 à 3 restes choisis dans le groupe des restes hydrogène, halogène, cyano, trifluorométhyle, trifluorométhoxy, nitro, alkyle en C₁ à C₄, alkoxy en C₁ à C₄, alkylthio en C₁ à C₄,
et
R³ représente l'hydrogène,
et leurs sels, leurs produits de solvatation et les produits de solvatation des sels.

4. Composés suivant les revendications 1, 2 ou 3, dans lesquels
R¹ est un reste 1-aza-bicyclo[2.2.2]oct-3-yle,
A est un groupe méthylène,
R² est un noyau benzothiophényle, quinolinyle ou naphtyle, les noyaux étant éventuellement substitués par 1 ou 2 restes choisis dans le groupe des restes hydrogène, fluor, chlore, brome, nitro,
et
R³ représente l'hydrogène,
et leurs sels, leurs produits de solvatation et les produits de solvatation des sels.

5. Procédé de production de composés de formule générale (I) suivant la revendication 1, **caractérisé en ce qu'**on fait réagir des composés de formule générale (II), 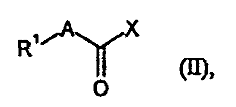 dans laquelle
R¹ et A ont la définition indiquée dans la revendication 1, et
X est un groupe hydroxy ou un groupe partant convenable,
avec un composé de formule générale (III)
R²R³NH (III),
dans laquelle R² et R³ ont la définition indiquée dans la revendication 1,
et on fait éventuellement réagir les composés (I) résultants avec (i) les solvants correspondants et/ou (ii) les bases ou les acides correspondants pour obtenir leurs produits de solvatation, leurs sels ou les produits de solvatation des sels.

6. Utilisation de composés suivant l'une des revendications 1 à 4, pour le traitement et/ou la prophylaxie de maladies.

7. Médicaments contenant au moins l'un des composés suivant l'une des revendications 1 à 4, en mélange avec au moins un support ou excipient essentiellement non toxique, pharmaceutiquement acceptable.

8. Utilisation de composés suivant l'une des revendications 1 à 4, pour la préparation d'un médicament destiné à améliorer la perception, la capacité de concentration, la capacité d'apprentissage et/ou la capacité de mémoire.

9. Utilisation de composés suivant l'une des revendications 1 à 4, pour la préparation d'un médicament destiné au traitement et/ou à la prophylaxie de troubles de la perception, de la capacité de concentration, de la capacité d'apprentissage et/ou de la capacité de mémoire.

10. Utilisation de médicaments suivant la revendication 7, pour le traitement et/ou la prophylaxie de troubles de la perception, de la capacité de concentration, de la capacité d'apprentissage et/ou de la capacité de mémoire.
